# EUROPÄISCHE PATENTSCHRIFT

(11) **EP 1 404 265 B1**
(45) Veröffentlichungstag und Bekanntmachung des Hinweises auf die Patenterteilung: **21.10.2009**
(21) Anmeldenummer: 02754760.3
(22) Anmeldetag: 26.06.2002
(51) Int. Cl.: A61F 9/009

(54) **VORRICHTUNG ZUR DARSTELLUNG EINES OPERATIONSGEBIETES BEI LASEROPERATIONEN**
DEVICE FOR REPRESENTING AN OPERATIVE FIELD DURING LASER OPERATIONS
DISPOSITIF PERMETTANT DE REPRESENTER UNE ZONE D'OPERATION LORS D'OPERATIONS PAR LASER.

(30) Priorität: 26.06.2001 DE 10130278
(43) Veröffentlichungstag der Anmeldung: 07.04.2004
(73) Patentinhaber: Carl Zeiss Meditec AG, 07745 Jena (DE)
(72) Erfinder: DICK, Manfred, 07926 Gefell (DE); KÜHNERT, Jürgen, 07749 Jena (DE); MÄUSEZAHL, Holger, 07745 Jena (DE)
(74) Vertreter: DTS München
(86) Internationale Anmeldenummer: PCT/EP2002/007073
(87) Internationale Veröffentlichungsnummer: WO 2003/002047

(56) Entgegenhaltungen:
- US-A- 5 098 426
- US-A- 6 099 522

## Beschreibung

Die vorliegende Erfindung betrifft ein Verfahren und eine Vorrichtung zur Darstellung eines Operationsgebietes bei Laseroperationen.

In der Ophthalmlogie ist es bekannt, die Hornhaut bei Sehschwäche durch Ablation von Gewebe zu formen. Als Verfahren haben sich die sogenannte PRK (Photo-Refraktive Keratektomie) und das LASIK-Verfahren LASIK (Laser assisted in situ Keratumilensis) etabliert, bei dem zunächst ein kleiner Lappen aus Ephitel, Bowman-Membran und Stroma geschnitten und aufgeklappt wird und sodann die PRK im Stromabett durchgeführt wird.

Zur Ablation werden Laser geeigneter Wellenlänge eingesetzt. Besonders geeignet hierzu ist der Excimerlaser mit einer Wellenlänge von 193 nm. Aber auch andere Laser wie Er: YAG-Festkörperlaser werden hierfür bereits eingesetzt.

Die Daten für das Ausmaß der Fehlsichtigkeit werden durch Sehtests, mittels Refraktometer und neuerdings auch durch Auswertung von Messungen der Wellenfront ermittelt. Es sind weiterhin auch Verfahren und Vorrichtungen bekannt, mittels derer aus diesen Messwerten Schusskoordinaten für den eigentlichen operativen Eingriff berechnet werden.

Die Verfolgung einer Laser-Operation durch den Operateur bzw. Arzt erfolgt überlicherweise durch Betrachtung des Operationsgebietes mittels eines Mikroskops. Hierbei erhält der Arzt einen räumlichen Eindruck vom Operationsgebiet. Allerdings erlaubt der Blick durch das Mikroskop nur einer Person einen räumlichen Eindruck.

Auch die Darstellung mittels Kamera auf einem Monitor etc. ist bekannt. Hierbei geht jedoch der räumliche Eindruck des Operationsgebietes verloren. Bei der Operation mittels Slitscanner kann durch den Arzt sehr gut der Fortschritt der Operation und die Reihenfolge der Ablationsschritte wahrgenommen bzw. überwacht werden. So kann der Arzt Abnormalitäten im Ablationsprozess frühzeitig erkennen und diesen gegensteuern, beispielsweise die Operation unterbrechen, etc.

Bei den heutigen modernen Spotscannern ist der Ablationsprozess aufgrund verschiedener Faktoren nicht mehr einfach nachvollziehbar. Zum Einen erhöht sich die Schussfrequenz der Laser permanent und liegt weit über der Wahrnehmungsmöglichkeit des Operateurs. Des Weiteren ist der Ablationsprozess patientenabhängig und die Ansteuerung und Platzierung des Spots im Ablationsgebiet unterliegt komplexen Algorithmen die unterschiedlichste Aufgaben und Probleme des Ablationsprozesses zu lösen versuchen (thermische Belastung, Rauch). Ein Ablationsalgorithmus zerlegt beispielsweise die notwendigen Schüsse für eine vollständige Korrektur in viele kleine Einzelkorrekturen mit dem Ziel, dass jederzeit die Operation abgebrochen werden kann und gewährleistet ist, dass ein optisch einwandfreies Ergebnis erreicht ist (vgl. den wertvollen Beitrag zum Stand der Technik in der Patentschrift DE 197 27 573). Daher ist eine Überwachung des komplexen Ablationsprozesses - durch Kontrolle der korrekten Positionierung des Laserstrahls auf dem zu bearbeitenden Gebiet - durch den Operateur bei moderneren Spotscannem durch den "Blick durch das Mikroskop" nicht mehr möglich.

Weiterhin ist das Sehfeld beim "Blick durch das Mikroskop" konstruktionsbedingt stark eingeschränkt. Um Operationsgeräte zu bedienen, Statusinformationen bzw. Ablaufinformationen von Geräten etc. zu erkennen, muss der Arzt den Blick durch das Operationsmikroskop verlassen und damit den Blick vom Operationsgebiet lösen.

Bei der Darstellung des Operationsgebietes für mehrere Betrachter (Lehrzwecke, Überwachung etc.) kann das Operationsgebiet mittels einer Kamera und eines Monitors dargestellt werden. Hierbei geht jedoch prinzipbedingt der räumliche Eindruck verloren. Dieser ist jedoch bei derart mikrochirurgischen Eingriffen von entscheidender Bedeutung.

Die Abtragsvorgänge bei der PRK mittels Spotscanner sind derart komplex, dass der Arzt kaum in der Lage ist, einen korrekten Operationsfortschritt zu erkennen bzw. eintretende Probleme frühzeitig zu erkennen, um korrigierend eingreifen zu können. Zur Gerätesteuerung etc. muss der Arzt den Blick vom Operationsgebiet entfernen, um die Geräte zu bedienen bzw. Statusinformationen aufzunehmen.

Die US 5 098 426 beschreibt ein Verfahren und eine Vorrichtung für die Präzisionslaserchirurgie vor.

Die US 6 099 522 beschreibt einen automatisierten Laserarbeitsplatz für chirurgische und industrielle Hochpräzisionslasereingriffe.

Der Artikel "Corneal Topography - The Dark Side of the Moon" von Leo D. Bores beschreibt Einzel-Seitenband-Holografie-Techniken zur genauen Vermessung der Oberfläche der Cornea.

Es ist deshalb Ziel und Aufgabe der Erfindung Vorrichtungen und Verfahren bereitzustellen, die es dem Arzt gestatten, den Operationsfortschritt eindeutig verfolgen zu können und somit die Operation bewusst zu kontrollieren.

Diese Aufgabe wird mit einer Vorrichtung nach dem unhabhängigen Vorrichtungsanspruch gelöst. Vorteilhafte Weiterbildungen sind in den abhängigen Ansprüchen definiert.

Insbesondere wird die Aufgabe durch eine Vorrichtung zur dreidimensionalen Darstellung eines Operationsgebietes, insbesondere eines Auges, bei Laseroperationen gelöst, umfassend ein räumliches Aufnahmesystem (10) ein Bildverarbeitungssystem (15) eine räumliche Anzeigeneinheit (20) ein Steuerungsmodul (30); mindestens ein medizinisches Gerät (40) und ein Bussystem, das die räumliche Anzeigeneinheit (20) und das Steuerungsmodul (30) verbindet, so dass Daten und Informationen auf der Anzeigeneinheit (20) angezeigt werden können.

Hierdurch ist es möglich, dass Zusatzinformationen der Operation bzw. des OP-Lasers etc. mit auf dem zu betrachtenden dreidimensionalen bzw. räumlichen Abbild des Operationsgebietes angezeigt werden können. Der Operateur und weitere Betrachter können so wertvolle Zusatzinformationen erfassen, ohne den Blick vom räumlich dargestellten Operationsgebiet nehmen zu müssen. Das Steuerungsmodul ist bevorzugt ein Computer, ein Rechner, ein Microkontroller.

Das medizinische Gerät umfasst bevorzugt einen OP-Laser zur refraktiven Chirurgie. Daneben können weitere Geräte wie Eye-Tracker, Online-Topografiesensor, Online-Wellenfrontsensor, Beleuchtungseinheiten sowie Augenidentifizierungssysteme (inclusive Rotationskontrolle und Identifizierung gemäß der Anmeldung DE 10052201 vom 20.10.2000) eingesetzt werden. Diese Geräte senden über das Bussystem Daten über den eigenen Status an die Steuereinheit und empfangen über das Bussystem auch Anweisungen vom Steuergerät. Besonders bevorzugt liegt der Algorithmus zum Abarbeiten des OP-Programmes sowie die Daten der geplanten Operation ebenfalls auf der Steuereinheit bzw. werden in diese eingespeist bzw. dieser zur Verfügung gestellt.

Daten und Informationen sind besonders bevorzugt Daten des Operationsgerätes, vorzugsweise eines Lasers zur refraktiven Hornhautchirurgie (wie Schußrate, Energie, Pulsform, etc.) bzw. Daten über die Operation (wie Patientendaten, Temperatur des Gewebes bzw. Operationsgebietes, bereits laufende Operationszeit, Restzeit der Operation, OP-Lasereinstellungen, etc.) oder Statusinformationen (Grenzen des Gebietes des Eyetrackers, Grenzen des Operationsbereiches des Lasers, Operationsfortschritt, Energiestatus, etc.). Durch diese Vorrichtung ist es möglich, das Operationsgebiet dreidimensional darzustellen und den Operationsfortschritt eindeutig zu verfolgen, selbst wenn man nicht durch das Mikroskop blickt. Insbesondere können auf diese Weise mehrere Betrachter das Operationsgebiet gleichzeitig betrachten, ohne den dreidimensionalen Eindruck zu verlieren.

Das räumliche Aufnahmesystem ist bevorzugt ein Kamerasystem, mit dem Bildinformationen bzw. -daten eines Objektes typischer Weise aus mehreren Blickrichtungen gewonnen werden können. Diese Daten ermöglichen dann die Rekonstruktion einer dreidimensionalen Abbildung dieses Objektes, d.h. des Operationsgebietes.

Das Bildverarbeitungssystem wertet die gewonnenen Daten aus und bereitet sie so auf, daß sie der nachfolgenden räumlichen Anzeigeeinheit zur Darstellung eines dreidimensionalen bzw. räumlichen Bildes genügen. Das Bildverarbeitungssystem kann ein Chip, ein Rechner, eine Software oder ein Mikroprozessor sein.

Die räumliche Anzeigeeinheit ist bevorzugt ein 3-D-Display, ein Hologramm oder eine durch Laser erzeugte dreidimensionale Darstellungseinheit. Besonders bevorzugt handelt es sich um eine Anordnung zur dreidimensionalen Darstellung gemäß DE 198 25 950. Mittels eines solchen Displays lässt sich in vorteilhafter Weise die räumliche Ausdehnung des Ausgangszustandes, d. h. des Zustandes vor dem operativen Eingriff, als auch die räumliche Ausdehnung des Zustandes nach einer Operation darstellen.

Bei einem weiteren bevorzugten Ausführungsbeispiel der vorliegenden Erfindung umfasst die erfindungsgemäße Vorrichtung weiterhin ein berührungssensitives Displayoverlay. Dadurch ist es für den Operateur möglich, Steuerbefehle bzw. Gerätesteuerungsfunktionen über das berührungssensitive Displayoverlay einzugeben. Diese können an die Steuereinheit weitergegeben und von dort zur Steuerung der medizinischen Geräte verwendet werden.

Bei einem weiteren bevorzugten Ausführungsbeispiel der vorliegenden Erfindung sind auf der Anzeigeneinheit (20) Bilddaten des Operationsgebietes, zusätzliche Daten und Informationen sowie Bedienfelder darstellbar. Durch diese Darstellung aller relevanten und interessanten Daten für die Operation ist es dem Operateur möglich, alle Parameter und Umstände der Operation im Auge zu behalten, ohne seinen Blick von dem räumlich dargestellten Operationsgebiet lösen zu müssen. Diese verschiedenen Daten können als PIP (picture in a picture) dargestellt werden oder überlagernd. Die Bilder und Daten können auch in einer Mehrfenstertechnik angezeigt werden.

Darüber hinaus ist es möglich, mehrere verschiedene Ansichten des Operationsgebietes gleichzeitig anzuzeigen. Dies können verschiedene Blickwinkel, verschiedene Perspektiven, Ausschnitte oder Vergrößerungsmaßstäbe sein. Daneben ist es möglich, Bilder verschiedener Zeitebenen nebeneinander darzustellen, was insbesondere dann bevorzugt ist, wenn der zeitliche Fortschritt einer Operation angezeigt werden soll. Dies ist insbesondere interessant für Lehrveranstaltungen, in denen die Daten und Verläufe von vorangegangenen Operationen vorgestellt werden sollen, ohne auf den räumlichen Eindruck zu verzichten.

Als Bedienfelder sind insbesondere Felder und Symbole vorgesehen, die durch Berühren des berührungssensitiven Displayoverlays aktiviert werden können. Bevorzugt sind dies Symbole zur Steuerung einer Vergrößerung des Bildes, insbesondere zur stufenlosen Vergrößerung (digitaler Zoom). Weiterhin bevorzugt sind Kameraführungssymbole zur Auswahl der Perspektive und des Betrachtungswinkels vorgesehen, Symbole zur Auswahl zwischen den einzelnen Fenstern, etc.

Ganz besonders bevorzugt ist eine Vorrichtung bei der Solldaten eines idealen Operationsgebietes nach der Operation, insbesondere einer idealen Cornea-Form, räumlich über einem Abbild des aktuellen Operationsgebietes, insbesondere der aktuellen Cornea, auf der räumlichen Anzeigeneinheit (20) darstellbar sind. Dadurch ist es möglich, die Daten der idealen Cornea über das Abbild der aktuellen, verkrümmten Cornea darzustellen und den Unterschied dadurch zu visualisieren. Während der Operation kann so direkt mitverfolgt werden, wie sich die bearbeitete Cornea immer mehr der dargestellten Idealform angleicht und schließlich in Deckung mit dieser gebracht wird. Zusätzlich können online ermittelte aktuelle Abweichungen der Cornea aufgrund einer Wellenfrontanalyse während der Operation aktuell mit den Anfangskorrekturwerten verglichen werden und so eine nochmals feinere Korrektur während des eigentlichen Operationsganges visualisiert werden.

Es werden damit Ausgangszustand und gewünschter Endzustand so darstellbar, dass die im Rahmen des operativen Eingriffs abzutragende Schichtdicke (Abtragsvolumen) ersichtlich ist.

Die erfindungsgemäße Vorrichtung besitzt weiterhin bevorzugt eine Simulationseinheit, die auf dem Display bei jedem einzelnen Laserschuss einen Abtrag realisiert, der in Ort und Volumenabtrag genau den Abtrag simuliert, der bei der Ablation auf der Cornea erfolgt. Die erfindungsgemäße Vorrichtung ist dabei so gestaltet, dass bei einer Operation bei jedem Laserschuss auf dem Display eine dem Volumenabtrag auf dem Auge adäquate Volumeneinheit auf dem Display entfernt wird. Mit der erfindungsgemäßen Vorrichtung lässt sich somit der Verlauf der Ablation der Cornea durch den Operateur verfolgen.

In einer bevorzugten Ausführung werden die Koordinaten für die Volumenentfernung auf dem Display für jeden einzelnen Schuss direkt am Scanner abgegriffen. Auf diese Art und Weise erfolgt der Abtrag am Display genau an der Stelle, die der jeweiligen Stellung der Scanner an dieser Stelle entspricht. Sollten die Scanner nicht die exakte Position anfahren, erfolgt auch am Display ein Abtrag mit entsprechend veränderten Koordinaten. Mittels der erfindungsgemäßen Vorrichtung ist somit eine wesentlich verbesserte Online-Kontrolle des Operationsverlaufes durch den Operateur möglich. Auch kann er am Ende der Operation den realisierten Endzustand in einfacher Art und Weise erfassen.

Sollte der realisierte Endzustand noch nicht ganz der gewünschten Korrektur entsprechen, kann der Operateur entscheiden, ob er sofort noch eine Nachkorrektur vornimmt. So lassen sich aus realisiertem Zustand und dem ursprünglich gewünschten Zustand weitere Koordinaten für die Ablation berechnen, so dass eine Nachkorrektur unmittelbar anschließend erfolgen kann.

Die Ermittlung von Koordinaten für eine Nachkorrektur erfolgt bevorzugt automatisch, so dass die Nachkorrektur sofort nach dem ursprünglichen Programm durchführbar ist. Die Verfolgung auch dieser Korrektur erfolgt gemäß dem Gedanken der Erfindung auf dem Display. So ist eine Online-Topografie der Corneaoberfläche auf der räumlichen Anzeigeeinheit darstellbar.

Bevorzugt ist die Simulationseinheit auch mit der Einheit zur Kontrolle der Laserenergie verbunden. Sollte die Laserenergie vom vorgesehenen Wert abweichen, kann auch der simulierte Volumenabtrag dementsprechend verändert werden.

Weiterhin kann die Simulationseinheit (Überwachungseinheit) an einen Online-Wellenfrontsensor angeschlossen werden. Die aktuelle Wellenfront des Auges wird räumlich dargestellt. Durch die räumliche Darstellung der momentanen Wellenfront des Auges während der Operation kann der Operateur den Fortschritt und Erfolg der Operation am Patienten unmittelbar verfolgen. Auch kann durch die Erfindung unmittelbar eine sich veränderte und abweichende Wellenfront von der idealen Wellenfront angezeigt werden. Diese kann durch unvorhersehbare Faktoren während der OP entstehen. Durch die räumliche Darstellung ist es dem Operateur somit möglich, eine fundierte Entscheidung zu treffen sich für eine weitere Korrektur unter anderen Parametern zu entscheiden (um auch die neue Wellenfrontaberrationen zu einer idealen Wellenfront zu führen) oder die Operation zu beenden.

Die erfindungsgemäße Vorrichtung kann insbesondere im Bereich der Opthalmologie eingesetzt werden. Beispielsweise kann mit der erfindungsgemäßen Vorrichtung die Materialbearbeitung bei Kontaklinsen oder Intraokularlinsen (IOL) verfolgt werden. Die Bearbeitung erfolgt dabei regelmäßig nicht am bzw. im Auge. Denkbar ist auch die Beobachtung des Bearbeitungsprozesses bei der Serienfertigung von Kontaktlinsen bzw. Intraokularlinsen. Hier ist insbesondere die Endzustandskontrolle der jeweiligen Linsen als Einsatzgebiet der erfindungsgemäßen Vorrichtung und des erfindungsgemäßen Verfahrens denkbar.

Möglich ist auch eine Verfolgung der Fertigung einer Linse, beispielsweise einer Intraokularlinse oder einer Kontaktlinse durch den Patienten selbst. Dieser kann sich auf diese Weise vorab ein Bild über die Erfolgschancen machen. Die Verfolgung des Bearbeitungsprozesses der individuellen Linse schafft Vertrauen beim Patienten für die sich anschließende Operation. Die bearbeitete Linse wird hierbei vorzugsweise in eine Zwischenbildebene eines optischen Systems eingespiegelt.

Eine weitere Einsatzmöglichkeit der erfindungsgemäßen Vorrichtung ist die Verfolgung der Materialbearbeitung an einer auf dem Auge befindlichen Kontaktlinse. Dabei kann das Gesamtsystem Auge-Kontaklinse gemeinsam vermessen werden. Die Bearbeitung erfolgt nur an der Kontaktlinse. Das Auge selbst wird nicht bearbeitet.

Die erfindungsgemäße Vorrichtung kann auch außerhalb medizinischer Anwendungen zum Einsatz gelangen. Grundsätzlich ist deren Verwendung bei jeder Art der Bearbeitung einer Oberfläche mittels eines Lasers möglich.

Die Erfindung soll im folgenden anhand von Zeichnungen weiter erläutert werden. Weitere vorteilhafte Merkmale sind hierbei beschrieben. Es zeigen:
- Fig. 1: eine schematische Darstellung einer erfindungsgemäßen Vorrichtung;
- Fig. 2: eine schematische Darstellung einer erfindungsgemäßen Vorrichtung zur Betrachtung für mehr als eine Person; und
- Fig. 3: eine Prinzip-Darstellung der Komponenten eines Ausführungsbeispieles einer Vorrichtung gemäß der vorliegenden Erfindung.

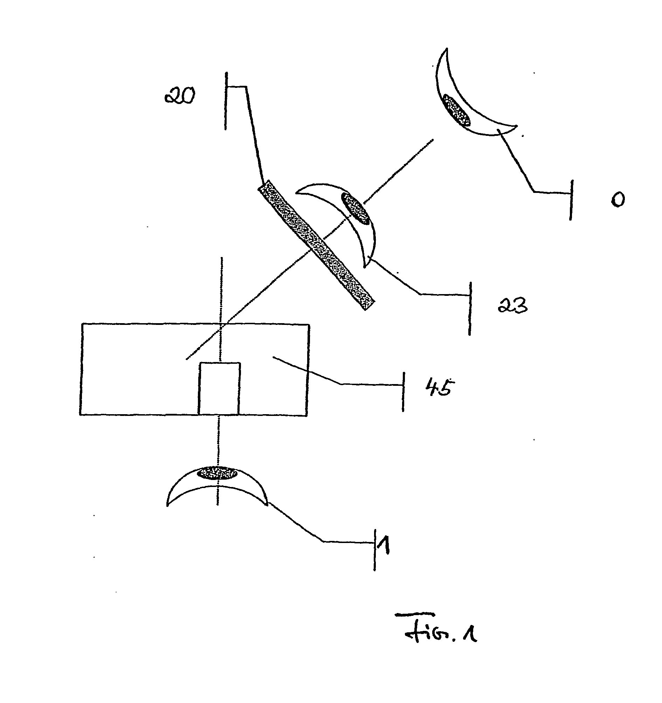

**Figur 1** zeigt eine schematische Darstellung einer erfindungsgemäßen Vorrichtung. Ein Operateur 0 betrachtet ein räumliches Bild 23 eines Auges eines Patienten, das durch ein Display zur räumlichen Darstellung 20 dargestellt wird. Das Auge des Patienten 1 wird dann mittels eines Lasers 45 bearbeitet.

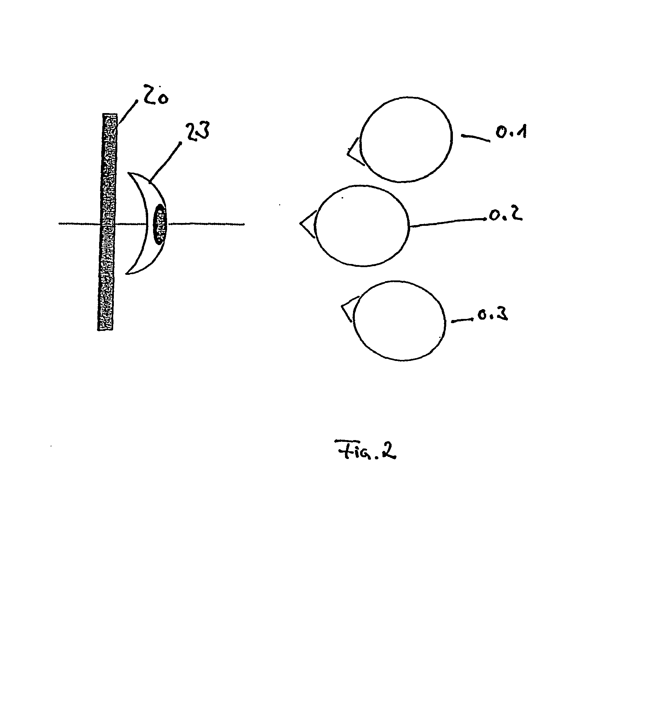

**Figur 2** zeigt eine schematische Darstellung einer erfindungsgemäßen Vorrichtung zur Betrachtung für mehr als eine Person. Mehrere Betrachter 0.1, 0.2 und 0.3 betrachten zeitgleich das räumliche Abbild des zu behandelnden Auges 23 mittels nur einer räumlichen Anzeigeeinheit 20.

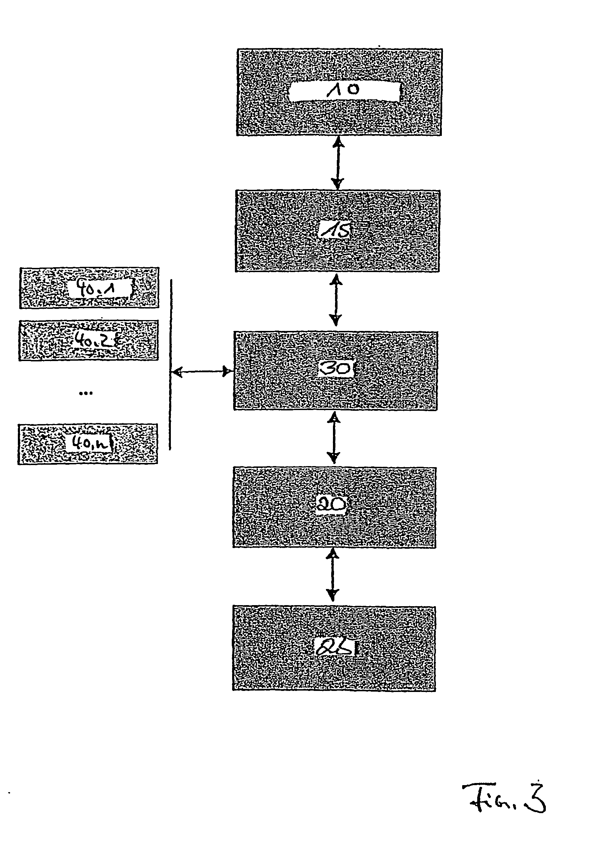

**Figur 3** zeigt eine Prinzip-Darstellung der Komponenten eines Ausführungsbeispieles einer Vorrichtung gemäß der vorliegenden Erfindung. Ein räumliches Aufnahmesystem 10 ist mit einem Bildverarbeitungssystem 15 verbunden. Dieses ist an ein Steuerungsmodul 30 angeschlossen. Das Steuerungsmodul 30 verbindet medizinische Geräte 40.1 bis 40.n mit der räumlichen Anzeigeeinheit 20. An der räumlichen Anzeigeeinheit 20 ist ein berührungssensitives Displayoverlay 25 angeschlossen.

Durch diesen erfindungsgemäßen Aufbau ist es möglich, ein Operationsgebiet durch das räumliche Aufnahmesystem 10 dreidimensional zu erfassen, diese Daten über ein Bildverarbeitungssystem 15 aufzubereiten und diese Informationen an die Steuereinheit 30 zu übergeben. Hier laufen auch die Informationen der medizinischen Geräte 40.n zusammen. Diese Daten werden dann gemeinsam oder alleine über die räumliche Anzeigeeinheit 20 dargestellt. Hier können sowohl das dreidimensionale Operationsgebiet als auch Statusdaten der medizinischen Geräte 40.n angezeigt werden. Über das berührungssensitive Displayoverlay 25 erhält der Operateur die Möglichkeit, Steuerbefehle einzugeben und so entweder Ansichten des Operationsgebietes auszuwählen (Mehrfenstertechnik, PIP, etc.), Statusdaten bzw. deren Verlauf abzufragen oder auch die medizinischen Geräte zu steuern.

Die Erfindung betrifft eine visuelle Vorrichtung zur räumlichen Darstellung des Operationsgebietes bei medizinischen Operationen - vorzugsweise bei Operationen am Auge mittels Laser zur Korrektur von Sehfehlern.

Mit der hier beschriebenen Erfindung ist es möglich, das Operationsgebiet auf einem Display sichtbar zu machen und es räumlich darzustellen. Durch die Erfindung ist es möglich, das Operationsgebiet unabhängig von einem Operationsmikroskop für eine Vielzahl von Betrachtern darzustellen. Das Operationsgebiet kann wesentlich größer dargestellt werden, es können beliebige Gebiete vergrößert werden bzw. simultan als PIP (Picture in Picture: Bild im Bild) dargestellt werden.

Neben der natürlicheren Darstellung des Operationsgebietes können simultan zur Operation Informationen eingeblendet bzw. dargestellt werden, die für den Arzt und den Operationsablauf von Bedeutung sind und so die vollständige Kontrolle des Gerätes ermöglichen ohne das Operationsgebiet aus dem Blick zu verlieren.

Durch eine zusätzliche Integration eines berührungsempfindlichen Displays kann somit die vollständige Ablaufsteuerung, Kontrolle des Gerätes etc. simultan erfolgen.

Die vorliegende Erfindung stellt somit eine Lösung dar die es ermöglicht, bei einer Operation das Operationsgebiet räumlich an einem Display für mehrere Personen darzustellen, beliebige Informationen in das Operationsgebiet einzublenden und das Operationsgerät - vorzugsweise ein Laser zur refraktiven Hornhautchirurgie - zu steuern.

**BEZUGSZEICHENLISTE**

| | |
|---|---|
| 0 | Beobachter |
| 1 | Operationsgebiet |
| 10 | Räumliches Aufnahmesystem |
| 15 | Bildbearbeitungssystem |
| 20 | Räumliche Anzeigeneinheit |
| 23 | Räumliches Abbild des Operationsgebietes |
| 25 | Berührungssensitives Displayoverlay |
| 30 | Steuerungsmodul |
| 40 | Medizinisches Gerät |
| 45 | Operationslaser |

## Patentansprüche

1. Vorrichtung zur dreidimensionalen Darstellung eines Operationsgebietes, insbesondere eines Auges, bei Laseroperationen, umfassend:
ein räumliches Aufnahmesystem (10),
ein Bildverarbeitungssystem (15),
eine räumliche Anzeigeneinheit (20) aus der Gruppe 3-D-Display, Hologramm oder durch Laser erzeugte dreidimensionale Darstellungseinheit,
ein Steuerungsmodul (30),
mindestens ein medizinisches Gerät (40), und
ein Bussystem, das die räumliche Anzeigeneinheit (20) und das Steuerungsmodul (30) verbindet, so dass Daten und Informationen auf der Anzeigeneinheit (20) an zeigt bar sind.

2. Vorrichtung gemäß Anspruch 1, wobei die Vorrichtung weiter eine Simulationseinheit umfasst, die auf der Anzeigeeinheit (20) bei jedem Laserschuss einen Abtrag simuliert, wobei die Koordinaten direkt am Scanner abgegriffen werden.

3. Vorrichtung nach einem der vorhergehenden Ansprüche,
**dadurch gekennzeichnet, dass**
zusätzlich ein berührungssensitives Displayoverlay vorgesehen ist.

4. Vorrichtung nach einem der vorhergehenden Ansprüche,
**dadurch gekennzeichnet, dass**
auf der Anzeigeneinheit (20) Bilddaten
des Operationsgebietes, zusätzliche Daten und Informationen sowie Bedienfelder darstellbar sind.

5. Vorrichtung nach einem der vorhergehenden Ansprüche,
**dadurch gekennzeichnet, dass**
Solldaten eines idealen Operationsgebietes nach der Operation, insbesondere einer idealen Cornea-Form, räumlich über einem Abbild des aktuellen Operationsgebietes, insbesondere der aktuellen Cornea, auf der räumlichen Anzeigeneinheit (20) darstellbar sind.

6. Vorrichtung nach einem der Ansprüche 2-5,
**dadurch gekennzeichnet, dass**
die Simulationseinheit mit einer Einheit zur Kontrolle der Laserenergie verbunden ist.

## Claims

1. Device for the three-dimensional representation of an operation area, in particular an eye, during laser operations, comprising
a spatial recording system (10),
an image-processing system (15),
a spatial display unit (20) of the group 3-D display, hologram or a three-dimensional representation unit generated by laser,
a control module (30),
at least one item of medical equipment (40), and
a bus system which connects the spatial display unit (20) and the control module (30), so that data and information are displayable on the display unit (20).

2. Device according to claim 1, whereas the device further comprises a simulation unit which, upon every single laser firing, realizes an ablation, at which the coordinates are measured directly from the scanner.

3. Device according to one of the preceding claims,
**characterized in that**
a touch-sensitive display overlay is additionally provided.

4. Device according to one of the preceding claims,
**characterized in that**
image data of the operation area, additional data and information and also control panels can be represented on the display unit (20).

5. Device according to one of the preceding claims,
**characterized in that**
reference data of an ideal operation area after the operation, in particular of an ideal cornea shape, can be represented on the spatial display unit (20) spatially via an image of the current operation area, in particular of the current cornea.

6. Method according to one of claims 2-5,
**characterized in that,**
the simulation unit is connected with a laser energy control unit.

## Revendications

1. Dispositif de représentation tridimensionnelle d'une zone d'opération, notamment d'un oeil, lors d'opérations par
laser, comprenant :
un système de prise de vue tridimensionnel (10) ;
un système de traitement d'image (15) ;
une unité d'affichage tridimensionnel (20) appartenant au groupe des écrans 3D, des hologrammes ou des unités de représentation à laser ;
un module de commande (30) ;
au moins un appareil médical (40) ; et
un système de bus qui relie l'unité d'affichage tridimensionnelle (20) et le module de commande (30) de façon à pouvoir afficher les données et informations sur l'unité d'affichage (20).

2. Dispositif selon la revendication 1, le dispositif comprenant en outre une unité de simulation simulant une ablation sur l'unité d'affichage (20) à chaque coup de laser, les coordonnées étant extraites directement au niveau du scanner.

3. Dispositif selon l'une quelconque des revendications précédentes, **caractérisé en ce qu'**un surécran tactile est également prévu.

4. Dispositif selon l'une quelconque des revendications précédentes, **caractérisé en ce que** des données d'image de la zone d'opération, des données supplémentaires ainsi que des informations et des champs d'utilisateur peuvent également être représentées sur l'unité d'affichage (20).

5. Dispositif selon l'une quelconque des revendications précédentes, **caractérisé en ce que** les données théoriques d'une zone d'opération idéale après l'opération, notamment une forme de cornée idéale, peuvent être représentées en trois dimensions sur l'unité d'affichage tridimensionnelle (20) par l'intermédiaire d'une image de la zone d'opération réelle, notamment de la cornée réelle.

6. Dispositif selon l'une quelconque des revendications 2 à 5, **caractérisé en ce que** l'unité de simulation est reliée à une unité de commande de l'énergie du laser.
